# EUROPÄISCHE PATENTSCHRIFT

(11) **EP 1 127 868 B1**
(45) Veröffentlichungstag und Bekanntmachung des Hinweises auf die Patenterteilung: **15.12.2004**
(21) Anmeldenummer: 01100548.5
(22) Anmeldetag: 10.01.2001
(51) Int. Cl.: C07C 67/36, C07C 69/38

(54) **Verfahren zur Herstellung von Malonsäurediestern in einem Reaktor mit innen liegenden Wärmeaustauschern**
Process for the preparation of malonic acid diesters in a reactor with an internal heat exchanger
Procédé de préparation de diesters d'acide malonique dans un réacteur à échangeur de chaleur interne

(30) Priorität: 25.02.2000 DE 10008903
(43) Veröffentlichungstag der Anmeldung: 29.08.2001
(73) Patentinhaber: Degussa AG, 40474 Düsseldorf (DE)
(72) Erfinder: Theis, Christoph, Dr., 53859 Niederkassel (DE); Bauer, Frank, Dr., 45721 Haltern (DE); Latz, Wilfried, 53844 Troisdorf (DE); Prange, Uwe, Dr., 53859 Niederkassel (DE)

(56) Entgegenhaltungen:
- EP-A- 0 633 060
- DE-A- 2 553 931
- DE-A- 19 836 807

## Beschreibung

Die Erfindung betrifft ein Verfahren zur Herstellung von Malonsäurediestern durch Carbonylierung von Halogenessigsäureestern, insbesondere Chloressigsäurealkylestern, mit Kohlenmonoxid und Umsetzung mit einwertigen Alkoholen und Basen in Gegenwart von Übergangsmetall-Katalysatoren unter Verwendung eines Reaktors mit einem oder mehreren innen liegenden Wärmeaustauscher(n) und einem Begasungsrührer.

Es ist bekannt, dass Malonsäurediester der allgemeinen Formel I 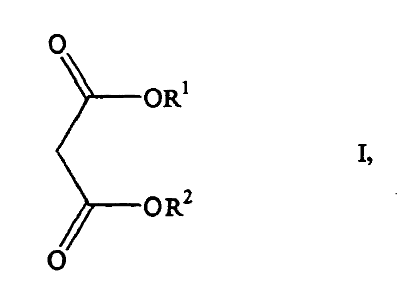 in der R¹ und R² jeweils unabhängig voneinander für eine unverzweigte oder verzweigte Alkyl- oder Alkenyl-, eine Cycloalkyl- oder eine Aralkylgruppe mit 1 bis 30 Kohlenstoffatomen, vorzugsweise für eine Alkyl-Gruppe mit 1 bis 6 Kohlenstoffatomen, steht durch Carbonylierung von Halogenessigsäureestern der allgemeinen Formel II 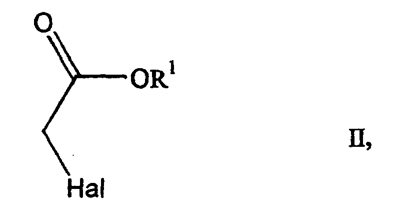 worin R¹ die vorstehende Bedeutung hat und Hal für ein Halogenatom steht, mit Kohlenmonoxid und Umsetzung mit einem einwertigen Alkohol der allgemeinen Formel R²OH, worin R² die vorstehende Bedeutung hat, vorzugsweise aber dem Rest R¹ in Formel II entspricht, unter Einsatz einer Base in Gegenwart eines Übergangsmetall-Katalysators hergestellt werden können.

Bei der Carbonylierung der Verbindungen der allgemeinen Formel II und Umsetzung mit einwertigen Alkoholen werden erhebliche Reaktionswärmen frei. Im technischen Maßstab wird die Reaktion daher üblicherweise in einem Schlaufenreaktor wie zum Beispiel einem so genannten BUSS-Reaktor durchgeführt (DE-OS 25 53 931).

Die erzielten Ausbeuten an den Verbindungen der allgemeinen Formel I liegen auch bei Realisierung der Reaktion im technischen Maßstab üblicherweise oberhalb von 90 % der Theorie bezogen auf die Menge an eingesetztem Halogenessigsäureester. Im Hinblick auf eine Minimierung der Produktionskosten gilt es daher insbesondere, die Raum-Zeit-Ausbeuten zu optimieren. Prinzipiell sollten sich diese durch Steigerung der Reaktionstemperatur und Erhöhen der Eduktkonzentrationen verbessern lassen.

Eine Steigerung der Reaktionstemperatur ist nicht nur wegen der damit verbundenen Erhöhung der Reaktionsgeschwindigkeit, sondern im Hinblick auf die hohe Wärmetönung der Reaktion auch wegen des größeren Temperaturunterschiedes zwischen Reaktionsmedium und Kühlmedium erstrebenswert.

Einer Erhöhung der Reaktionstemperatur sind aber aus mehreren Gründen Grenzen gesetzt. So verbieten sich Reaktionstemperaturen deutlich oberhalb von 100 °C schon deshalb, weil der Katalysator im Allgemeinen dann selbst in Gegenwart hoher Kohlenmonoxid-Partialdrücke nicht mehr stabil ist.

Auch ist bekannt, dass die Ausbeute an den Malonsäurediestern, bezogen auf die umgesetzte Menge an Halogenessigsäureestern, mit zunehmender Reaktionstemperatur abnimmt. So liegen die isolierten Ausbeuten an dem besonders bedeutenden Malonsäuredimethylester um 2 bis 3 Prozent niedriger, wenn die Reaktion unter ansonsten unveränderten Bedingungen bei 90 °C anstatt bei 50 °C bis 70 °C durchgeführt wird. Umgekehrt sind Reaktionstemperaturen deutlich unterhalb von 90 °C im technischen Maßstab wegen der damit verbundenen erheblichen Verlängerungen der Reaktionszeiten (JP 57-183 741) nicht akzeptabel.

Eine Erhöhung der Eduktkonzentration unterliegt ebenfalls Einschränkungen. So fallen die während der Reaktion gebildeten Halogenide in der Regel als feste Salze an. Auch handelt es sich vielfach bei den eingesetzten Basen schon um unter den Reaktionsbedingungen kristalline Feststoffe (zum Beispiel Natriumcarbonat). Der Salzanfall führt - insbesondere zusammen mit dem während der Reaktion gebildeten Wasser - bei vergleichsweise hohen Eduktkonzentrationen von beispielsweise 25 % Feststoffanteil in der Reaktionsmischung dazu, dass diese bei Durchführung der Reaktion in einem Rührreaktor oder Schlaufenreaktor beziehungsweise BUSS-Reaktor nicht mehr völlig gleichmäßig durchmischt werden kann. Auch wurde gefunden, dass eine Steigerung des Gehaltes der Reaktionsmischung an zum Beispiel Chloressigsäurealkylester und/oder Malonsäuredialkylester über 3,75 Mol/l Reaktionsvolumen hinaus in herkömmlichen Reaktoren mit einer Verminderung der Selektivität einhergeht.

Zwar bestehen Möglichkeiten, die Selektivität der Carbonylierungsreaktion durch Zusätze zur Reaktionsmischung zu verbessern (JP 54 112 818), jedoch kann dadurch die Aufarbeitung der Reaktorausträge erschwert werden. Auch sind im Hinblick auf eine Verwertung des/der Lösemittel(s), des Katalysators beziehungsweise dessen Folgeprodukten und vor allem des Salzanfalls Verunreinigungen durch Zusätze von erheblichem Nachteil.

Es bestand somit die Aufgabe, ein Verfahren zur Herstellung von Malonsäurediestern der allgemeinen Formel I durch Carbonylierung von Halogenessigsäureestern der allgemeinen Formel II mit Kohlenmonoxid und Umsetzung mit einwertigen Alkoholen aufzufinden, welches die oben genannten Nachteile nicht aufweist und welches verbesserte Raum-Zeit-Ausbeuten bei gleichzeitig unverminderten oder verbesserten Produktselektivitäten liefert.

Überraschend wurde nun gefunden, dass sehr hohe Raum-Zeit-Ausbeuten erzielt werden können, wenn die Carbonylierungsreaktion und Umsetzung mit dem einwertigen Alkohol in einem Rührreaktor mit einem oder mehreren innen liegenden Wärmeaustauscher(n) durchgeführt wird und der Rührreaktor einen Begasungsrührer enthält.

Gegenstand der Erfindung ist daher ein Verfahren zur Herstellung von Malonsäurediestern der allgemeinen Formel I, 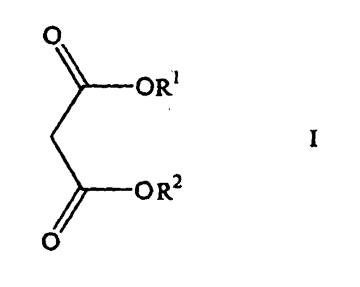 in der R¹ und R² jeweils unabhängig voneinander für eine unverzweigte oder verzweigte Alkyl- oder Alkenyl-, eine Cycloalkyl- oder eine Aralkylgruppe mit 1 bis 30 Kohlenstoffatomen steht, durch Carbonylierung von Halogenessigsäureestern gemäß Formel II, 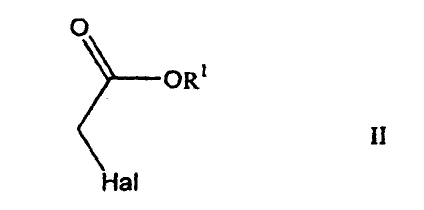 worin R¹ die vorstehende Bedeutung hat und Hal für ein Halogenatom steht, unter Einsatz von Kohlenmonoxid, eines einwertigen Alkohols der allgemeinen Formel R²OH, worin R² die vorstehende Bedeutung hat, einer Base und eines Übergangsmetall-Katalysators, dadurch gekennzeichnet, dass die Reaktion in einem Rührreaktor mit einem oder mehreren innen liegenden Wärmeaustauscher(n) durchgeführt wird und der Rührreaktor einen Begasungsrührer enthält.

Auf diese Weise konnten zum Beispiel zur Herstellung des technisch besonders bedeutenden Malonsäuredimethylesters bis zu 5,2 Mol Chloressigsäuremethylester pro Liter am flüssigen Anteil des Reaktionsgemisches eingesetzt werden, ohne dass es zu Durchmischungsproblemen kam. Auch waren die so erzielten Ausbeuten an isoliertem Zielprodukt (Gehalt: > 99,7 %) von beispielsweise 92,0 % mit denjenigen vergleichbar, welche unter analogen Bedingungen - aber in größerer Verdünnung - im sogenannten BUSS-Reaktor (91,5 %) beziehungsweise Rührreaktor (91,3 %) erhalten wurden.

Die Komponenten können bei Umgebungstemperaturen (Raumtemperaturen) zusammengegeben werden. Die Reaktionstemperaturen betragen 40 bis 100 °C, vorzugsweise 50 bis 95 °C. Unabhängig vom verwendeten Reaktortyp erwies es sich jedoch als vorteilhaft, die im Hinblick auf hohe Raum-Zeit-Ausbeuten erstrebenswerten hohen Reaktionstemperaturen von beispielsweise 90 °C kontinuierlich über einen definierten Temperaturgradienten anzusteuern. Als besonders vorteilhaft erwies es sich dabei, das Reaktionsgemisch zunächst auf die zum Starten der Reaktion erforderliche Temperatur von beispielsweise 50 °C zu erwärmen, bevor die Temperatur dann schrittweise oder - bevorzugt - kontinuierlich auf beispielsweise 90 °C gesteigert wurde. Gegebenenfalls kann dann eine Nachreaktionsphase auf demselben oder einem niedrigeren Temperaturniveau folgen.

Um im Falle des Rührreaktors und des Rührreaktors mit einem oder mehreren innen liegenden Wärmetauscher(n) eine gute Durchmischung des Kohlenmonoxids mit der Suspension aus den übrigen Komponenten der Reaktionsmischung zu ermöglichen, wird ein Begasungsrührer verwendet. Auf diese Weise kann auch im technischen Maßstab eine ausreichende Verteilung des Kohlenmonoxids in der Reaktionsmischung erreicht werden, ohne dass externe Pumpen beziehungsweise Kompressoren eingesetzt werden müssen.

Überraschend hat sich weiterhin gezeigt, dass Anbackungen des während der Reaktion gebildeten Halogenids weitestgehend vermieden werden können, wenn ein Rührreaktor mit Begasungsrührer und innen liegenden Wärmeaustauschern gemäß der EP-A-0 633 060 - im weiteren Text auch als "BIAZZI"-Reaktor bezeichnet - eingesetzt wird.

Im Hinblick auf die hervorragende Durchmischung des Kohlenmonoxids mit den übrigen Komponenten der Reaktionsmischung im Schlaufenreaktor beziehungsweise "BUSS"-Reaktor wurde bei Verwendung eines "BIAMI"-Reaktors für die Carbonylierung der Halogenessigsäureester und Umsetzung mit dem einwertigen Alkohol darüber hinaus noch überraschend gefunden, dass der Katalysator bei gleichem Kohlenmonoxid-Partialdruck und gleicher Temperatur einer deutlich geringeren Zersetzungsrate unterliegt. Mithin können höhere Reaktionstemperaturen realisiert werden und/oder es kann eine geringere als die übliche Menge an Übergangsmetall-Katalysator eingesetzt werden, wodurch sich schließlich geringere Kosten für dessen Rückführung in den Prozess ergeben.

Das Halogen in dem Halogenessigsäureester steht für Chlor, Brom oder Jod. Vorzugsweise werden Chloressigsäureester eingesetzt.

Als Übergangsmetall-Katalysatoren können Übergangsmetall-Komplexe oder Übergangsmetall-Komplexsalze mit Übergangsmetallen, ausgewählt aus der Gruppe, die durch Cobalt, Ruthenium, Palladium und Platin gebildet wird, eingesetzt werden. Cobalt wird als Übergangsmetall bevorzugt. Als Cobaltcarbonyl-Komplex-Katalysator wird vorzugsweise das Dicobaltoctacarbonyl und hieraus generierbare Spezies wie zum Beispiel Alkalisalze, insbesondere Natriumsalze, des Cobaltcarbonylwasserstoffs eingesetzt
Als Basen können insbesondere Alkali- und Erdalkalihydroxide, -carbonate undhydrogencarbonate eingesetzt werden. Die Natriumverbindungen, insbesondere das Natriumcarbonat, werden dabei bevorzugt.

Ebenfalls überraschend wurde gefunden, dass die isolierten Ausbeuten an den Malonsäurediestern der allgemeinen Formel I und mithin die erzielten Selektivitäten - unabhängig vom verwendeten Reaktortyp - ansteigen, wenn die Reaktionsmischung neben dem Halogenessigsäureester der allgemeinen Formel II, dem einwertigen Alkohol der allgemeinen Formel R²OH, dem Kohlenmonoxid, der Base und dem Übergangsmetall-Katalysator 0,1 bis 60 Gew.-%, bevorzugt 5 bis 40 Gew.-%, besonders bevorzugt 10 bis 30 Gew.-%, jeweils bezogen auf die gesamte Reaktionsmischung, eines unpolaren, unter den Reaktionsbedingungen inerten Lösemittels (Cosolvens) enthält. Als besonders vorteilhaftes Cosolvens hat sich Toluol erwiesen.

Mit steigendem Toluol-Zusatz ergeben sich zunächst steigende isolierte Ausbeuten an den Verbindungen der allgemeinen Formel I. Schließlich wird aber ein Punkt maximaler Selektivität erreicht, oberhalb dessen die isolierten Ausbeuten an den Verbindungen der allgemeinen Formel I stark abfallen.

Mit den isolierten Ausbeuten einher geht das rheologische Verhalten der Reaktionsmischung. Während geringe Toluolzusätze erwartungsgemäß einen geringen Einfluß auf die Rheologie der Reaktionsmischung haben, führen Zusätze von > 60 % Toluol, bezogen auf die gesamte Reaktionsmischung, bei Durchführung der Reaktion im Rührreaktor oder im Schlaufenreaktor beziehungsweise so genannten BUSS-Reaktor zur Bildung des Reaktionssalzes in zunehmend schmieriger, schwer zu handhabender und zu durchmischender Form. Darüber hinaus kommt es zu Anbackungen des Reaktionssalzes, welche durch Spülen mit dem eingesetzten Alkohol oder Alkohol/Cosolvens-Gemisch nur noch teilweise entfernt werden können.

Überraschenderweise können bei Durchführung der Reaktion im "BIAZZI"-Reaktor größere Cosolvens-Mengen in der Reaktionsmischung enthalten sein, ohne dass die genannten Probleme auftreten. Mit diesen höheren Cosolvens-Gehalten gehen wiederum überraschend höhere isolierte Ausbeuten an den Malonsäurediestern der allgemeinen Formel I und somit höhere Selektivitäten einher. Daher können bei Durchführung der Carbonylierungsreaktionen und Umsetzungen mit dem einwertigen Alkohol im "BIAZZI"-Reaktor unter Cosolvens-, insbesondere Toluol-Zusatz, trotz erhöhter Edukt-Konzentrationen und der damit verbundenen Steigerung der Raum-Zeit-Ausbeuten höhere isolierte Ausbeuten von zum Beispiel bis zu 94,3 % der Theorie für den Malonsäuredimethylester (Gehalt: > 99,8 %) erzielt werden.

Der Katalysator wird nach Beendigung der Reaktion vorzugsweise durch Sauerstoff oder ein sauerstoffhaltiges Gas zersetzt.

Als grundsätzlicher Vorteil eines Rührreaktors - ob ohne oder mit einem oder mehreren innen liegenden Wärmetauschern - gegenüber einem Schlaufenreaktor beziehungsweise "BUSS"-Reaktor ist die Möglichkeit einer schnelleren Entspannung der Gasphase des Reaktors anzusehen. Die in der Reaktionsmischung gelösten Mengen an während der Reaktion gebildetem Kohlendioxid bewirken bei schneller Entspannung ein starkes Schäumen, welches bei kleinen Gas-Flüssigkeits-Grenzflächen zum Mitreißen von flüssigen und festen Komponenten der Reaktionsmischung führt.
Zusammen mit den oben dargestellten Ergebnissen zur Steigerung der Raum-Zeit-Ausbeute des Verfahrens ergeben sich daher deutlich verkürzte Taktzeiten im Falle einer diskontinuierlichen (batch)-Fahrweise des Reaktors beziehungsweise es sind im Vergleich zum einfachen Rührreaktor und dem Schlaufen- beziehungsweise "BUSS"-Reaktor höhere Durchsätze bei einem kontinuierlichen Reaktorbetrieb möglich. Erfindungsgemäß kann so die Zeit für das Befüllen und Entleeren des "BIAZZI"-Reaktors, das Aufheizen der Reaktionsmischung bis zum Beginn der Kohlenmonoxid-Aufnahme sowie die eigentliche Carbonylierungsreaktion und Umsetzung mit dem einwertigen Alkohol auf Taktzeiten deutlich weniger als 120 Minuten, vorzugsweise nicht mehr als 90 Minuten, reduziert werden.

Die Reaktionszeit, gemessen vom Beginn der Kohlenmonoxid-Aufnahme bis zum Erzielen eines Halogenessigsäureester-Umsatzes von > 99,8 % beträgt vorzugsweise nicht mehr als 90 Mintuten.

Malonsäurediester sind vielseitige Synthese-Bausteine in der organischen Chemie zum Beispiel als Zwischenprodukte bei der Synthese von Pharmaka, Kunststoffen, Pflanzenschutzmitteln, Riechstoffen, Aromen und Farbstoffen.

Die folgenden Beispiele sollen das erfindungsgemäße Verfahren weiter erläutern, ohne dessen Anwendungsbereich einzuschränken.

### Beispiel 1: Herstellung von Malonsäuredimethylester (Vergleichsbeispiel Rührreaktor)

In einem 2-Liter-Rührautoklaven wurde eine Mischung aus 542,2 g Chloressigsäuremethylester, 727,4 g Methanol, 293,1 g Natriumcarbonat und 16,3 g Dicobaltoctacarbonyl unter Rühren bei Raumtemperatur vorgelegt. Dann wurde die Mischung unter einem Kohlenmonoxid (CO)-Partialdruck von 25 bar schrittweise auf schließlich 92 °C aufgeheizt, wobei der Temperaturanstieg so gewählt wurde, dass eine Überhitzung des Reaktionsgemisches ausgeschlossen war.
Die Reaktionszeit, gemessen vom Beginn der Kohlenmonoxid-Aufnahme bis zum Erzielen eines Chloressigsäuremethylester-Umsatzes von > 99,7 %, betrug 90 Minuten.
Die isolierte Ausbeute an Malonsäuredimethylester betrug nach wässriger Aufarbeitung des Reaktionsgemisches 91,3 % der Theorie, bezogen auf die Menge an eingesetztem Chloressigsäuremethylester. Der so hergestellte Malonsäuredimethylester wies eine gaschromatographisch bestimmte Reinheit von > 99,5 % auf.

### Beispiel 2: Herstellung von Malonsäuredimethylester (Vergleichsbeispiel "BUSS"-Reaktor)

Es wurde wie in Beispiel 1 verfahren, als Reaktor kam jedoch ein "BUSS"-Reaktor zum Einsatz. Bedingt durch das Reaktorprinzip musste die Reaktion hierbei in größerem Maßstab als in Beispiel 1 durchgeführt werden.
Die Reaktionszeit, gemessen vom Beginn der Kohlenmonoxid-Aufnahme bis zum Erzielen eines Chloressigsäuremethylester-Umsatzes von > 99,5 %, betrug 150 Minuten.
Die isolierte Ausbeute an Malonsäuredimethylester betrug 91,5 % der Theorie, bezogen auf die Menge an eingesetztem Chloressigsäuremethylester. Der so hergestellte Malonsäuredimethylester wies eine gaschromatographisch bestimmte Reinheit von > 99,5 % auf

### Beispiel 3: Herstellung von Malonsäuredimethylester unter Verwendung eines "BIAZZI"-Reaktors.

Es wurde wie in Beispiel 1 verfahren, als Reaktor kam jedoch ein "BIAZZI"-Reaktor zum Einsatz. Bedingt durch das Reaktorprinzip musste die Reaktion in größerem Maßstab als in Beispiel 1 durchgeführt werden.
Die Reaktionszeit, gemessen vom Beginn der Kohlenmonoxid-Aufnahme bis zum Erzielen eines Chloressigsäuremethylester-Umsatzes von > 99,7 %, betrug 70 Minuten.
Die isolierte Ausbeute an Malonsäuredimethylester betrug 93,4 % der Theorie, bezogen auf die Menge an eingesetztem Chloressigsäuremethylester. Der so hergestellte Malonsäuredimethylester wies eine gaschromatographisch bestimmte Reinheit von > 99,8 % auf.

### Beispiel 4: Herstellung von Malonsäuredimethylester ("BIAZZI"-Reaktor)

Es wurde wie in Beispiel 3 verfahren, die Einsatzstoffmenge an Chloressigsäuremethylester wurde jedoch auf 4,8 Mol pro Liter Reaktionsvolumen gesteigert.

Darüber hinaus wurde ein Toluol-Gehalt der Reaktionsmischung von 17,1 Gew.-% eingestellt.

Die Gesamtzeit für das Befüllen und Entleeren des Reaktors sowie die eigentliche Reaktionszeit, gemessen vom Beginn der Kohlenmonoxid-Aufnahme bis zum Erzielen eines Chloressigsäuremethylester-Umsatzes von > 99,8 %, betrug 90 Minuten.
Der nach Beendigung der Reaktion erhaltene Reaktoraustrag enthielt das Reaktionssalz in leicht dispergierbarer, keineswegs schmieriger Form. An den Reaktorwandungen anhaftendes Reaktionssalz konnte auch nach mehreren Versuchen problemlos durch Spülen mit Methanol/Toluol-Gemisch vollständig entfernt werden.
Die isolierte Ausbeute an Malonsäuredimethylester betrug 92,0 % der Theorie, bezogen auf die Menge an eingesetztem Chloressigsäuremethylester. Der so hergestellte Malonsäuredimethylester wies eine gaschromatographisch bestimmte Reinheit von > 99,8 % auf

### Beispiel 5: Herstellung von Malonsäuredimethylester (Rührreaktor) (Vergleichsbeispiel)

Es wurde wie in Beispiel 4 verfahren, als Reaktor kam jedoch ein einfacher Rührreaktor zum Einsatz.
Der nach Beendigung der Reaktion erhaltene Reaktoraustrag enthielt das Reaktionssalz in schmieriger, schwierig zu handhabender Form. An den Reaktorwandungen anhaftendes Reaktionssalz konnte nach mehreren Versuchen durch Spülen mit Methanol oder Methanol/Toluol-Gemisch nicht mehr vollständig entfernt werden.
Die isolierte Ausbeute an Malonsäuredimethylester betrug lediglich 87 % der Theorie, bezogen auf die Menge an eingesetztem Chloressigsäuremethylester. Der so hergestellte Malonsäuredimethylester wies eine gaschromatographisch bestimmte Reinheit von > 99 % auf.

## Patentansprüche

1. Verfahren zur Herstellung von Malonsäurediestern der allgemeinen Formel I, 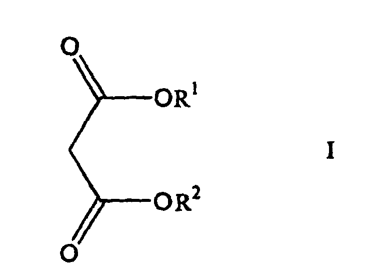 in der R¹ und R² jeweils unabhängig voneinander für eine unverzweigte oder verzweigte Alkyl- oder Alkenyl-, eine Cycloalkyl- oder eine Aralkylgruppe mit 1 bis 30 Kohlenstoffatomen steht, durch Carbonylierung von Halogenessigsäureestern der allgemeinen Formel II, 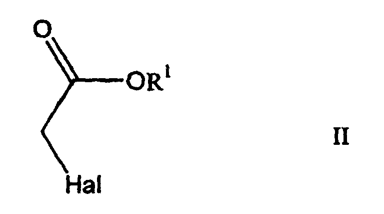 worin R¹ die vorstehende Bedeutung hat und Hal für ein Halogenatom steht, unter Einsatz von Kohlenmonoxid und Umsetzung mit einem einwertigen Alkohol der allgemeinen Formel R²OH, worin R² die vorstehende Bedeutung hat, und einer Base in Gegenwart eines Übergangsmetall-Katalysators,
**dadurch gekennzeichnet, dass**
die Reaktion in einem Rührreaktor mit einem oder mehreren innen liegenden Wärmetauscher(n) durchgeführt wird und der Rührreaktor einen Begasungsrührer enthält.

2. Verfahren nach Anspruch 1,
**dadurch gekennzeichnet, dass**
die Reaktionstemperatur mittels eines vorgewählten Temperaturgradienten bis zur Endtemperatur gesteigert wird.

3. Verfahren nach einem der Ansprüche 1 bis 2,
**dadurch gekennzeichnet, dass**
die Reaktionsmischung zusätzlich 0,1 bis 60 Gew.-% eines unpolaren unter den Reaktionsbedingungen inerten Lösemittels enthält.

4. Verfahren nach Anspruch 3,
**dadurch gekennzeichnet, dass**
als unpolares Lösemittel Toluol verwendet wird.

5. Verfahren nach einem der Ansprüche 1 bis 4,
**dadurch gekennzeichnet, dass**
die Reaktionszeit, gemessen vom Beginn der Kohlenmonoxid-Aufnahme bis zum Erzielen eines Halogenessigsäureester-Umsatzes von > 99,8 %, nicht mehr als 90 Minuten beträgt.

6. Verfahren nach einem der Ansprüche 1 bis 5,
**dadurch gekennzeichnet, dass**
als Halogenessigsäureester Chloressigsäureester eingesetzt wird.

7. Verfahren nach einem der Ansprüche 1 bis 6,
**dadurch gekennzeichnet, dass**
der Übergangsmetall-Katalysator nach Beendigung der Reaktion durch Sauerstoff oder ein sauerstoffhaltiges Gas zersetzt wird.

8. Verfahren nach einem der Ansprüche 1 bis 7,
**dadurch gekennzeichnet, dass**
das Übergangsmetall des Übergangsmetall-Katalysators aus der Gruppe, die durch Cobalt, Ruthenium, Platin und Palladium gebildet wird, ausgewählt wird.

9. Verfahren nach Anspruch 8,
**dadurch gekennzeichnet, dass**
der Katalysator Cobalt als Übergangsmetall enthält.

10. Verfahren nach Anspruch 9,
**dadurch gekennzeichnet, dass**
der Übergangsmetall-Katalysator Dicobaltoctacarbonyl als Einsatzmaterial enthält.

## Claims

1. A process for preparing malonic diesters of the formula I, 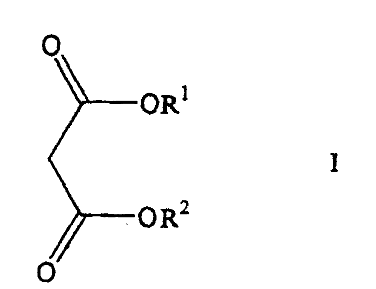 where R¹ and R² are each, independently of one another, an unbranched or branched alkyl or alkenyl group, a cycloalkyl group or an aralkyl group having from 1 to 30 carbon atoms, by carbonylation of haloacetic esters of the formula II, 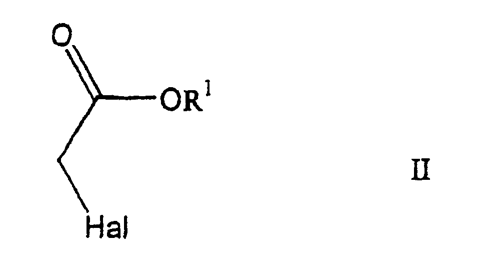 where R¹ is as defined above and Hal is a halogen atom, using carbon monoxide and reaction with a monohydric alcohol of the formula R²OH, where R² is as defined above, and a base in the presence of a transition metal catalyst, **characterized in that** the reaction is carried out in a stirred reactor with one or more internal heat exchangers and the stirred reactor contains a sparging stirrer.

2. A process according to claim 1, **characterized in that** the reaction temperature is increased to the final temperature by means of a preselected temperature gradient.

3. A process according to either one of claims 1 and 2, **characterized in that** the reaction mixture further comprises from 0.1 to 60% by weight of a nonpolar solvent which is inert under the reaction conditions.

4. A process according to claim 3, **characterized in that** the nonpolar solvent used is toluene.

5. A process according to any one of claims 1 to 4, **characterized in that** the reaction time, measured from the .beginning of carbon monoxide absorption to the achievement of a haloacetic ester conversion of > 99.8%, is not more than 90 minutes.

6. A process according to any one of claims 1 to 5, **characterized in that** the haloacetic ester used is a chloroacetic ester.

7. A process according to any one of claims 1 to 6, **characterized in that** the transition metal catalyst is decomposed after the reaction is complete by means of oxygen or an oxygen-containing gas.

8. A process according to any one of claims 1 to 7, **characterized in that** the transition metal of the transition metal catalyst is selected from the group consisting of cobalt, ruthenium, platinum and palladium.

9. A process according to claim 8, **characterized in that** the transition metal present in the catalyst is cobalt.

10. A process according to claim 9, **characterized in that** the transition metal catalyst comprises dicobalt octacarbonyl as starting material.

## Revendications

1. Procédé de préparation de diesters d'acide malonique de formule générale I 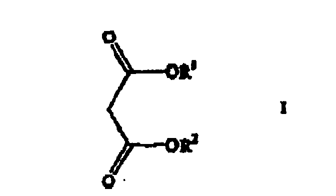 dans laquelle R¹ et R², chacun indépendamment l'un de l'autre, représentent un groupe non ramifié ou ramifié alkyle ou alcényle, cycloalkyle ou aralkyle portant de 1 à 30 atomes de carbone, par carboxylation d'esters de l'acide acétique halogéné de formule générale II, 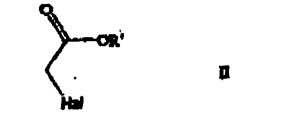 dans laquelle R¹ a la signification précédente et Hal représente un atome d'halogène, avec utilisation de monoxyde de carbone et mise en réaction avec un alcool monovalent de formule générale R²OH, dans laquelle R² a la signification précédente, et d'une base en présence d'un catalyseur de métal de transition,
**caractérisé en ce que**
la réaction est réalisée dans un réacteur agité ayant un ou plusieurs échangeurs de chaleur internes et le réacteur agité renferme un agitateur de gazage.

2. Procédé selon la revendication 1,
**caractérisé en ce que**
la température de réaction est augmentée jusqu'à une température finale au moyen d'un gradient de température présélectionné.

3. Procédé selon l'une quelconque des revendications 1 à 2,
**caractérisé en ce que**
le mélange réactionnel contient en plus 0,1 à 60 % en poids d'un solvant apolaire, inerte dans les conditions de la réaction.

4. Procédé selon la revendication 3,
**caractérisé en ce que**
comme solvant apolaire on utilise du toluène.

5. Procédé selon l'une quelconque des revendications 1 à 4,
**caractérisé en ce que**
le temps de réaction, mesuré depuis le début de l'absorption de monoxyde de carbone jusqu'à l'obtention d'une conversion de l'ester d'acide acétique halogéné supérieure à 99,8 %, ne dépasse pas 90 minutes.

6. Procédé selon l'une quelconque des revendications 1 à 5,
**caractérisé en ce que**
comme ester de l'acide acétique halogéné, on met en oeuvre l'ester de l'acide acétique chloré.

7. Procédé selon l'une quelconque des revendications 1 à 6,
**caractérisé en ce que**
le catalyseur de métal de transition est décomposé, à l'issue de la réaction, par de l'oxygène ou un gaz contenant de l'oxygène.

8. Procédé selon l'une quelconque des revendications 1 à 7,
**caractérisé en ce que**
le métal de transition du catalyseur métal de transition est choisi dans le groupe constitué par le cobalt, le ruthénium, le platine et le palladium.

9. Procédé selon la revendication 8,
**caractérisé en ce que**
le catalyseur contient du cobalt en tant que métal de transition.

10. Procédé selon la revendication 9,
**caractérisé en ce que**
le catalyseur de métal de transition contient du dicobalt octacarbonyle en tant que matière de mise en oeuvre.
